(19) Europäisches Patentamt
European Patent Office
Office européen des brevets

(11) Publication number: 0 336 068 A1

# (12) EUROPEAN PATENT APPLICATION

(21) Application number: 89101886.3

(22) Date of filing: 03.02.89

(51) Int. Cl.4: C07D 311/66 , A61K 31/35

The title of the invention has been amended (Guidelines for Examination in the EPO, A-III, 7.3).

(30) Priority: 11.02.88 US 154765

(43) Date of publication of application: 11.10.89 Bulletin 89/41

(84) Designated Contracting States: AT BE CH DE FR GB IT LI LU NL SE

(71) Applicant: F. HOFFMANN-LA ROCHE AG
Postfach 3255
CH-4002 Basel(CH)

(72) Inventor: Laurenzano, Anthony James
6 Marston Drive
Morris Plains, N.J. 07950(US)
Inventor: Partridge, John Joseph
620 Airport Road
Chapel Hill North Carolina 27514(US)

(74) Representative: Kellenberger, Marcus, Dr. et al
Grenzacherstrasse 124 Postfach 3255
CH-4002 Basel(CH)

## (54) Phenoxyalkoxy-3,4-dihydro-2H-1-benzopyran derivatives.

(57) Compounds of the formula

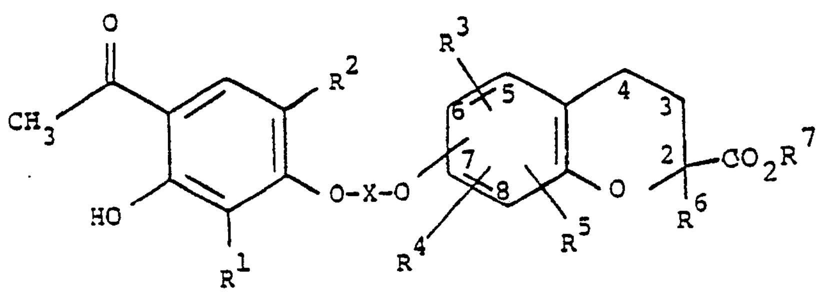

I

wherein $R^1$ is hydrogen or lower alkyl, $R^2$ is hydrogen or halogen, $R^3$, $R^4$ and $R^5$, independently, are hydrogen, acyl or lower alkyl, provided that only one of $R^3$, $R^4$ and $R^5$ can be acyl, $R^6$ is hydrogen or lower alkyl, $R^7$ is higher alkyl or benzyl and X is $(C_{3-7})$-alkylene,
as well as enantiomes thereof, are useful for treating allergic and inflammatory disorders. They can be prepareed according to known methods.

EP 0 336 068 A1

# Phenoxyalkoxy-3,4-dihydro-24-1-benzopyran derivatives

The present invention relates to compounds of the general formula

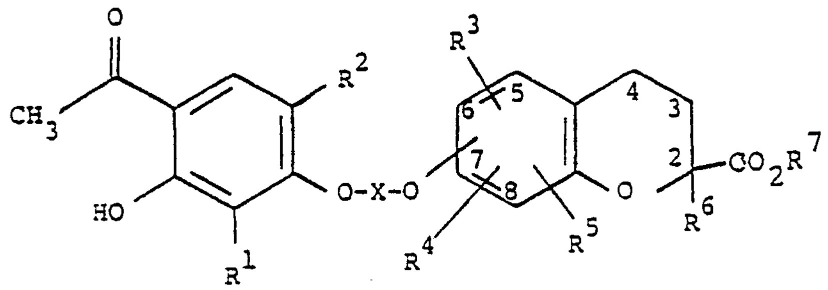

I

wherein $R^1$ is hydrogen or lower alkyl, $R^2$ is hydrogen or halogen, $R^3$, $R^4$ and $R^5$, independently, are hydrogen, acyl or lower alkyl, provided that only one of $R^3$, $R^4$ and $R^5$ can be acyl, $R^6$ is hydrogen or lower alkyl, $R^7$ is higher alkyl or benzyl and X is $(C_{3-7})$-alkylene,
as well as enantiomers thereof.

The compounds of formula I are useful as agents for the treatment of allergic and inflammatory disorders, including dermatological conditions such as psoriasis, contact dermatitis and eczema, as well as inflammatory bowel disease.

As used herein, the term "higher alkyl" refers to a straight or branched chain saturated hydrocarbon group containing 8 to 20 carbon atoms, for example, n-octyl, iso-octyl (2-methylheptyl), 3-methylheptyl, 4-methylheptyl, n-nonyl, 2-methyloctyl, 3-methyloctyl, 4-methyloctyl, n-decyl, 2-methylnonyl, 3-methylnonyl, 4-methylnonyl, 5-methylnonyl, 2-ethyloctyl, 3-ethyloctyl, 4-ethyloctyl, n-undecyl, 2-methyldecyl, 3-methyldecyl, 4-methyldecyl, 5-methyldecyl, 2-ethylnonyl, 3-ethylnonyl, 4-ethylnonyl, 5-ethylnonyl, n-dodecyl, 2-methyldecyl, 3-methyldecyl, 4-methyldecyl, 5-methyldecyl, n-tetradecyl, n-hexadecyl and the like.

The term "lower alkyl" denotes a straight or branched chain saturated hydrocarbon group containing 1 to 7 carbon atoms, for example, methyl, ethyl, propyl, isopropyl, butyl, i-butyl, t-butyl, neopentyl, pentyl, heptyl and the like.

The term "halogen" refers to the halogens bromide, chlorine, fluorine and iodine.

The term "acyl" refers to a lower alkanoyl group derived from an aliphatic carboxylic acid of 1 to 7 carbon atoms, for example, formyl, acetyl, propionyl and the like; and an arylcarbonyl group derived from an aromatic carboxylic acid, such as benzoyl and the like.

The term "$(C_{3-7})$-alkylene" refers to a straight or branched chain radical having from 3 to 7 carbon atoms, for example, propylene, 2-methylpropylene, butylene, pentamethylene, hexamethylene, heptamethylene and the like.

The term "aryl" refers to phenyl or phenyl bearing one or more substituents selected from the group consisting of halogen, trifluoromethyl, lower alkyl, lower alkoxy, lower thioalkoxy, nitro, amino, lower alkyamino and di-lower alkylamino.

The term "aralkyl" refers to a straight or branched chain lower alkyl group in which one or more of the hydrogen atoms have been replaced by an aryl group, for example, benzyl and the like.

An especially preferred group of compounds of the invention are those represented by the formula

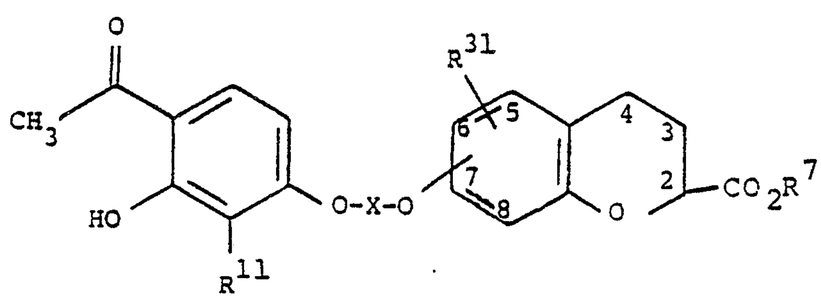

Ia

wherein $R^{11}$ is lower alkyl and $R^{31}$ is acyl and $R^7$ and X are as above,

and enantiomers thereof.

A still more preferred group of compounds of the invention are those of formula Ia in which $R^{11}$ is propyl, $R^{31}$ is acetyl in 6-position, $R^7$ is higher alkyl or benzyl and X is $(C_{4-6})$-alkylene.

Particularly preferred compounds of formula I are :

Racemic 6-acetyl-7-[5-(4-acetyl-3-hydroxy-2-propylphenoxy)pentyloxy]-3,4-dihydro-2H-1-benzopyran-2-carboxylic acid n-decyl ester,
racemic 6-acetyl-7-[5-(4-acetyl-3-hydroxy-2-propylphenoxy)pentyloxy]-3,4-dihydro-2H-1-benzopyran-2-carboxylic acid n-dodecyl ester,
racemic 6-acetyl-7-[5-(4-acetyl-3-hydroxy-2-propylphenoxy)pentyloxy]-3,4-dihydro-2H-1-benzopyran-2-carboxylic acid n-hexadecyl ester,
racemic 6-acetyl-7-[5-(4-acetyl-3-hydroxy-2-propylphenoxy)pentyloxy]-3,4-dihydro-2H-1-benzopyran-2-carboxylic acid benzyl ester,
(R)-(-)-6-acetyl-7-[5-(4-acetyl-3-hydroxy-2-propylphenoxy)pentyloxy]-3,4-dihydro-2H-1-benzopyran-2-carboxylic acid n-octyl ester and
(S)-(+)-6-acetyl-7-[5-(4-acetyl-3-hydroxy-2-propylphenoxy)pentyloxy]-3,4-dihydro-2H-1-benzopyran-2-carboxylic acid n-octyl ester.

The most preferred compound of formula I is:
Racemic 6-acetyl-7-[5-(4-acetyl-3-hydroxy-2-propylphenoxy)pentyloxy]-3,4-dihydro-2H-1-benzopyran-2-carboxylic acid n-octyl ester.

In accordance with the present invention the compounds of formula I can be prepared by a process which comprises

a) reacting a compound of the general formula

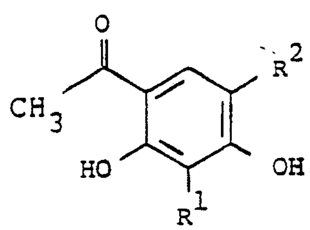 II or 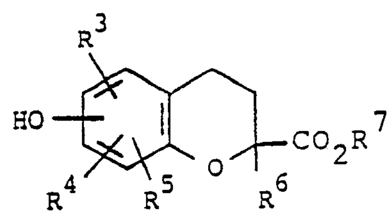 III

wherein $R^1$, $R^2$, $R^3$, $R^4$, $R^5$, $R^6$ and $R^7$ are as above,
with, respectively, a compound of the general formula

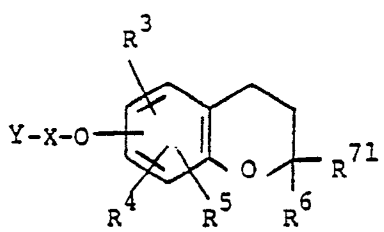 IV and 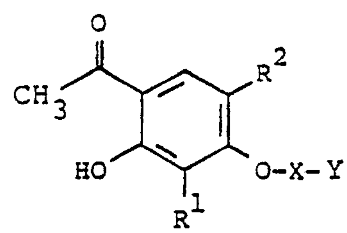 V

wherein $R^1$, $R^2$, $R^3$, $R^4$, $R^5$, $R^6$, $R^7$ and X are as above and Y is halogen, alkylsulfonate, aralkylsulfonate or arylsulfonate,

b) esterifying a compound of the general formula

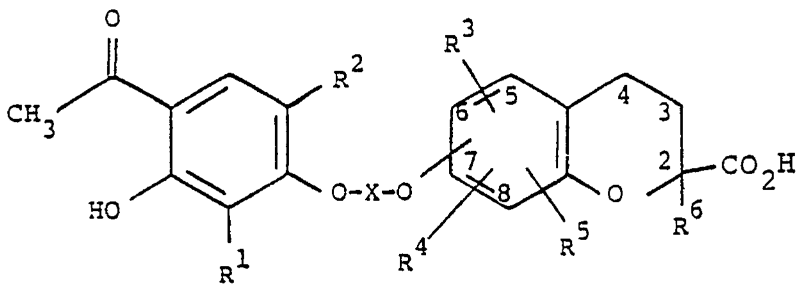

VI

wherein $R^1$, $R^2$, $R^3$, $R^4$, $R^5$, $R^6$ and X are as above,
and

c) if desired, resolving a racemate obtained into the optically active isomers.

The reaction of a compound of formula II with a compound of formula IV and, respectively, the reaction of a compound of formula III with a compound of formula V in accordance with process aspect a) to yield a compound of formula I can be carried out under anhydrous conditions in an inert solvent, for example, acetone, methyl ethyl ketone, diethyl ketone, ethyl acetate, acetonitrile, toluene, dimethylformamide or the like, at the reflux temperature of the reaction mixture, preferably at a temperature in the range of from about 60° to about 120° C, and in the presence of an acid acceptor, for example, potassium carbonate or the like. The preferred solvent is a mixture of acetone and dimethylformamide, or toluene alone in the presence of a phase transfer agent such as tris[2-(2-methoxyethoxy)ethyl]amine. The resulting compound of formula I can be recovered utilizing conventional methods, for example, crystallization, chromatography, or the like.

In acordance with process aspect b) a compound of formula VI can be reacted with a higher alkyl or benzyl alcohol ($R^7OH$) in an inert solvent, such as methylene chloride, 1,2-dichloroethane, benzene or toluene, preferably toluene, in the presence of a strong acid, such as methanesulfonic acid or p-toluenesulfonic acid monohydrate. The reaction can be carried out under anhydrous conditions, at the reflux temperature of the reaction mixture, preferably at a temperature in the range of from about 40° to about 120° C. The resulting compound of formula I can be recovered utilizing conventional methods, for example, crystallization, chromatography, or the like.

The compounds of formulae II, III, IV, V and VI used as starting materials are known compounds or can be prepared according to known procedures such as those described in the European Patent Publication 0.129.906.

The compounds of formula I possess an asymmetric carbon atom and are ordinarily obtained as racemic mixtures. The resolution of such racemates into the optically active isomers can be carried out by known procedures. Some racemic mixtures can be precipitated as eutectics and can thereafter be separated. However, chemical resolution at an earlier stage of the synthesis is preferred. By this method, diastereomers are formed, for example, from the racemic mixture of a compound of formula VI with an optically active resolving agent, for example, an optically active base such as R-(+)- or S-(-)-α-methylbenzylamine, R-(+)- or S-(-)-1-(1-napthyl)ethylamine, quinine, quinidine, ephedrine and the like, which can be reacted with a carboxyl group. The formed diastereomers salts are separated by selective crystallization and converted to the corresponding optical isomers. Thus, this invention encompasses the racemates of formula I, as well as their optically active isomers (enantiomers).

Alternatively, resolution can be achieved by treatment of an acid corresponding to formula IV or formula III, but wherein $R^7$ is hydrogen with an optically active amine resolving agent such as R-(+)- or S-(-)-α-methylbenzylamine, R-(+)- or S-(-)-1-(1-napthyl)ethylamine, quinine, quinidine, ephedrine and the like. After separation of he diastereomeric salts as described above, the optically active carboxylic acid obtained is recovered by strong acid treatment. The optically active carboxylic acid corresponding to formula IV or formula III but wherein $R^7$ is hydrogen is then converted into the optically active ester of, respectively, formula IV and formula III which is then converted into the corresponding optically active compound of formula I, as described for the preparation of the racemic series.

In addition, resolution can be achieved by treatment of an acid corresponding to formula IV or formula III but wherein $R^7$ is hydrogen with an optically active alcohol to produce a mixture of diastereomeric esters which can be separated by crystallization or chromatography. Representative optically active alcohol resolving agents are menthol, borneol, 2-octanol, 2,3-butanediol and the like. The preferred mode of resolution involves the mono-ester of 2R,3R-butanediol and acids derived from esters of formula IV and formula III. These esters can be formed using conventional methods. The preferred method is conventional Fisher esterfication using a strong acid catalyst. The preferred acid catalyst is p-toluenesulfonic acid

monohydrate. The preferred chromatographic technique for achieving the resolution is high performance liquid chromatography (HPLC).

The useful pharmacological activity of the compounds of formula I can be demonstrated in warm-blooded animals utilizing standard pharmacological procedures. An exemplary of such procedures is:

Antibiotic-Induced Colitis in Hamsters (In Vivo):

Male Syrian hamsters (LUG), weighing 80 to 120 g, were each given a single dose of 175 mg/kg of clindamycin-phosphate or of clindamycin-hydrochloride intraperitoneally, to induce colitis. Approximately seven hours after injection, the animals wer given the test compound orally or intraperitoneally and the therapy was continued twice a day for a period of four more days. The effect of the therapy was measured by use of the Hazard Ratio, which is the ratio of the mortality of the animals treated with the test compound contained in a vehicle to the mortality of the animals treated with the vehicle containing none of the test compound. The mortality was determined for the test compound-treated groups and for the vehicle-treated groups, respectively, twice daily, and was evaluated by comparing the survival curves of each group. The Kaplan-Meier estimate of the survival curve was calculated for each group and the Mantel-Cox (logrank) test was used to compare the survival curve of each test compound-treated (therapy) group to that of the corresponding vehicle control group. A Hazard Ratio of 1.0 indicates that the therapy has no better effect compared with the vehicle alone, while a Hazard Ratio greater than 1.0 (>1.0) indicates that the therapy prolongs survival in comparison with the group treated with the vehicle alone.

Racemic 6-acetyl-7-[5-(4-acetyl-3-hydroxy-2-propylphenoxy)pentyloxy]-3,4-dihydro-2H-1-benzopyran-2-carboxylic acid n-octyl ester was found to be active when given orally at 20 micromoles per kilogram, exhibiting a Hazard Ratio of 2.0.

A compound of formula I or a composition containing a therapeutically effective amount of a compound of formula I can be administered by methods well known in the art. Thus, the compounds of the present invention can be administered either singly or with other pharmaceutical agents, for example, antihistamines, mediator release inhibitors, methyl xanthines, beta-agonists or antiasthmatic steroids such as prednisone and prednisolone, orally, parenterally, rectally or by inhalation, for example, in the form of an aerosol, micropulverized powder or nebulized solution. For oral administration they can be administered in the form of tablets, capsules, for example, in admixture with talc, starch, milk sugar or other inert ingredients, that is, pharmaceutically acceptable carriers, or in the form of aqueous solutions, suspensions, elixirs or aqueous alcoholic solutions, for example, in admixture with sugar or other sweetening agents, flavoring agents, colorants, thickeners and other conventional pharmaceutical excipients. For parenteral administration, they can be administered in solutions or suspension, for example, as an aqueous or peanut oil solution or suspension using excipients and carriers conventional for this mode of administration. For administration as aerosols, they can be dissolved in a suitable pharmaceutically acceptable solvent, for example, ethyl alcohol or combinations of miscible solvents, and mixed with a pharmaceutically acceptable propellant. Such aerosol compositions are packaged for use in a pressurized container fitted with an aerosol valve suitable for release of the pressurized composition. Preferably, the aerosol valve is a metered valve, that is one which on activation releases a predetermined effective dose of the aerosol composition.

Presently, the most preferred route of administration for the compounds of formula I is the oral route, for example, as a tablet, and particularly for use as an agent against inflammatory bowel disease.

In the practice of the invention, the dose of a compound of formula I to be administered and the frequency of administration will be dependent on the potency and duration of activity of the particular product to be administered and on the route of administration, as well as the severity of the condition, age of the warm-blooded animal to be treated and the like. Doses of a compound of formula I contemplated for use in the practice of the invention are in the range of from about 25 to 1000 mg per day, preferably about 25 to about 250 mg either as a single dose or in divided doses pe day.

The Examples which follow further describe the invention.

## Example 1

Preparation of racemic 6-acetyl-7-[5-(4-acetyl-3-hydroxy-2-propylphenoxy)pentyloxy]-3,4-dihydro-2H-1-benzopyran-2-carboxylic acid n-octyl ester

A 1 L flask equipped with a mechanical stirrer, a Dean-Stark trap, a reflux condenser and a calcium chloride drying tube, was charged with 50.0 g (0.100 mole) of racemic 6-acetyl-7-[5-(4-acetyl-3-hydroxy-2-propylphenoxy)pentyloxy]-3,4-dihydro-2H-1-benzopyran-2-carboxylic acid, 13.0 g (0.100 mole) of 1-octanol, 0.5 g (0.0025 mole) of p-toluenesulfonic acid monohydrate and 500 ml of toluene. The mixture was heated at the reflux temperature for 2 hours under an inert atmosphere with removal of water. After cooling to room temperature, the brown solution was washed successively with 2 x 100 ml = 200 ml of brine solution, 3 x 100 ml = 300 ml of saturated aqueous sodium bicarbonate solution and 2 x 100 ml = 200 ml of brine solution. The organic phase was dried over anhydrous sodium sulfate, filtered and evaporated to dryness under reduced pressure at 35 mm and 45° C, and finally at high vacuum (0.1 mm, 45° C), to give a brown semi-solid residue, This residue was triturated with 500 ml of boiling ether and chilled (12° C) in the refrigerator overnight to effect crystallization. Filtration and drying at room temperature in a vacuum oven (70 mm Hg) gave 46.0 g (75% yield) of racemic 6-acetyl-7-[5-(4-acetyl-3-hydroxy-2-propylphenoxy)-pentyloxy]-3,4-dihydro-2H-1-benzopyran-2-carboxylic acid n-octyl ester as an off-white solid, m.p. 81-84° C.

## Example 2

Preparation of racemic 6-acetyl-7-[5-(4-acetyl-3-hydroxy-2-propylphenoxy)pentyloxy]-3,4-dihydro-2H-1-benzopyran-2-carboxylic acid n-dodecyl ester

A 1 L flask equipped with a mechanical stirrer, a Dean-Stark trap, a reflux condenser and a calcium chloride drying tube, was charged with 30.0 g (0.060 mole) of racemic 6-acetyl-7-[5-(4-acetyl-3-hydroxy-2-propylphenoxy)pentyloxy]-3,4-dihydro-2H-1-benzopyran-2-carboxylic acid 11.2 g (0.060 mole) of 1-dodecanol, 1.0 g (0.005 mole) of p-toluenesulfonic acid monohydrate and 300 ml of toluene. The mixture was heated at reflux temperature for 1.5 hours under an inert atmosphere with removal of water. After cooling to room temperature, the brown solution was washed successively with 2 x 60 ml = 120 ml of brine solution, 3 x 60 ml = 180 ml of saturated aqueous sodium bicarbonate solution, and 2 x 60 ml = 120 ml of brine solution. The organic phase was dried over anhydrous sodium sulfate, filtered and evaporated to dryness under reduced pressure at 35 mm and 45° C, and finally at high vacuum (0.1 mm, 45° C), to give 36.8 of a dark brown oil. This oil was dissolved in 50 ml of ether and passed through a plug column containing 100 g of silica gel (70-230 mesh, Fluka) with 800 ml of ether eluant. The ether eluate was concentrated at 70 mm to a volume of 400 ml. Crystallization was induced by the addition of 40 ml of hexanes and chilling at -20° C overnight. The solid was collected by filtration to yield 28.0 (70% yield) of racemic 6-acetyl-7-[5-(4-acetyl-3-hydroxy-2-propylphenoxy)pentyloxy]-3,4-dihydro-2H-1-benzopyran-2-carboxylic acid n-dodecyl ester as an off-white solid, m.p. 46-48° C.

## Example 3

Preparation of racemic 6-acetyl-7-[5-(4-acetyl-3-hydroxy-2-propylphenoxy)pentyloxy]-3,4-dihydro-2H-1-benzopyran-2-carboxylic acid n-hexadecyl ester

A 500 ml flask equipped with a mechanical stirrer, a Dean-Stark trap, a reflux condenser and a calcium chloride drying tube, was charged width 25.0 (0.050 mole) of racemic 6-acetyl-7-[5-(4-acetyl-3-hydroxy-2-propylphenoxy)pentyloxy]-3,4-dihydro-2H-1-benzopyran-2-carboxylic acid 11.5 g (0.047 mole) of 1-hexadecanol, 0.8 g (0.0042 mole) of p-toluenesulfonic acid monohydrate and 250 ml of toluene. The mixture was heated at the reflux temperature for 2 hours under an inert atmosphere with removal of water. After cooling to room temperature, the solution was washed consecutively with 2 x 60 ml = 120 ml of brine solution, 3 x 60 ml = 180 ml of saturated aqueous sodium bicarbonate solution, and 2 x 60 ml = 120 ml of brine solution. The organic phase was dried over anhydrous sodium sulfate, filtered and evaporated to dryness under reduced pressure at 35 mm and 45° C, and finally at high vacuum (0.1 mm, 45° C), to give 31.7 of a brown oil. This residue was dissolved in 100 ml of hexanes at reflux and chilled in a refrigerator overnight at 12° C to effect crystallization. The solid was filtered, washed with 2 x 20 ml = 40 ml of cold (12° C) hexanes and air dried to give 29.4 g (86.5% yield) of racemic 6-acetyl-7-[5-(4-acetyl-3-hydroxy-2-propylphenoxy)pentyloxy]-3,4-dihydro-2H-1-benzopyran-2-carboxylic acid n-hexadecyl ester as an off-white solid, m.p. 58-60° C.

## Example 4

Preparation of racemic 6-acetyl-7-[5-(4-acetyl-3-hydroxy-2-propylphenoxy)pentyloxy]-3,4-dihydro-2H-1-benzopyran-2-carboxylic acid benzyl ester

A 2 L flask equipped with a mechanical stirrer, a Dean-Stark trap, a reflux condenser and a calcium chloride drying tube, was charged with 50.0 g (0.100 mole) of racemic 6-acetyl-7-[5-(4-acetyl-3-hydroxy-2-propylphenoxy)pentyloxy]-3,4-dihydro-2H-1-benzopyran-2-carboxylic acid 10.5 g (0.100 mole) of benzyl alcohol 1.0 g (0.005 mole) of p-toluenesulfonic acid monohydrate and 1.0 L of toluene. The mixture was heated at reflux temperature for 2 hours under an inert atmosphere with removal of water. After cooling to room temperature, the brown solution was washed consecutively with 2 x 500 ml = 1.0 L of brine solution, 3 x 500 ml = 1.5 ml of aqueous sodium bicarbonate solution, and 2 x 500 ml = 1.0 L of brine solution. The organic phase was dried over anhydrous sodium sulfate, filtered and evaporated to dryness under reduced pressure at 35 mm and 45° C, and finally at high vacuum (0.1 mm, 45° C), to give 52.0 g of a brown oil. The oil was dissolved in 350 ml of ether at room temperature and allowed to crystallize in the refrigerator overnight at (12° C). Filtration and drying in a vacuum oven (70 mm Hg) at room temperature overnight gave 36.4 g (62.% yield) of racemic 6-acetyl-7-[5-(4-acetyl-3-hydroxy-2-propylphenoxy)pentyloxy]-3,4-dihydro-2H-1-benzopyran-2-carboxylic acid benzyl ester as an off-white solid, m.p. 108-110° C.

## Example 5

Preparation of (R)-(-)-6-acetyl-7-[5-(4-acetyl-3-hydroxy-2-propylphenoxy)pentyloxy]-3,4-dihydro-2H-1-benzopyran-2-carboxylic acid n-octyl ester

A 250 ml flask equipped with a Dean-Stark trap, a mechanical stirrer, a reflux condenser and a calcium chloride drying tube, was charged with 8.0 g (0.016 mole) of R-(-)-6-acetyl-7-[5-(4-acetyl-3-hydroxy-2-propylphenoxy)pentyloxy]-3,4-dihydro-2H-1-benzopyran-2-carboxylic acid, 2.8 g (0.016 mole) of 1-octanol, 0.5 g (0.0026 mole) of p-toluenesulfonic acid monohydrate and 150 ml of toluene. The mixture was heated at reflux temperature for 2 hours with the removal of water. After cooling to room temperature, the solution was washed with 2 x 50 ml = 100 ml of brine, 3 x 50 ml = 150 ml of saturated sodium bicarbonate solution, and 2 x 50 ml = 100 ml of brine. The organic phase was dried over anhydrous sodium sulfate, filtered and evaporated under reduced pressure at 40° C (70 mm) and at 40° C (1mm) to give 9.0 g of an amber oil. This oil was chromatographed on a low pressure (3 psi) flash column containing 100 g of silica gel G60 (Fluka, 70-230 mesh) using 5:1 hexanes-ethyl acetate as the mobile phase to give 7.3 g of a light yellow oil residue. This oil was crystallized by dissolving in 30 ml of ether, adding 30 ml of hexanes, seeding and chilling in the refrigerator (7° C) overnight. Filtration and drying in a vacuum oven to constant weight gave 3.1 g (32% yield) of (R)-(-)-6-acetyl-7-[5-(4-acetyl-3-hydroxy-2-propylphenoxy)pentyloxy]-3,4-dihydro-2H-1-benzopyran-2-carboxylic acid n-octyl ester as a white solid, m.p. 47-60° C; $[\alpha]_D^{25}$ -1.81° (c, 0.9940, $CHCl_3$).

## Example 6

Preparation of (S)-(+)-6-acetyl-7-[5-(4-acetyl-3-hydroxy-2-propylphenoxy)pentyloxy]-3,4-dihydro-2H-1-benzopyran-2-carboxylic acid n-octyl ester

A 100 ml flask equipped with a Dean-Stark trap, a mechanical stirrer, a reflux condenser and a calcium

chloride drying tube, was charged with 1.5 g (0.003 mole) of S-(+)-6-acetyl-7-[5-(4-acetyl-3-hydroxy-2-propylphenoxy)pentyloxy]-3,4-dihydro-2H-1-benzopyran-2-carboxylic acid, 0.39 g (0.003 mole) of 1-octanol, 0.10 g (0.0005 mole) of p-toluenesulfonic acid monohydrate and 30 ml of toluene. The mixture was heated at reflux temperature for 2 hours with the removal of water. After cooling to room temperature, the brown solution was washed successively with 2 x 30 ml = 60 of brine, 3 x 30 ml = 90 ml of saturated sodium bicarbonate solution, and 2 x 30 ml = 60 ml of brine solution. The organic phase was dried over anhydrous sodium sulfate, filtered and evaporated to dryness at 40° C (1 mm) to give 1.9 g of orange oil. This oil was combined with 0.5 of similar quality ester and purified on a Waters Prep 500 High Pressure Liquid Chromatograph using 1:45 ethyl acetate-hexanes as the mobile phase, to give 1.9 of a light yellow oil. This oil was crystallized from a 1:1-mixture of ether and hexane to give 0.50 g (20.4% yield) of off-white solid (S)-(+)-6-acetyl-7-[5-(4-acetyl-3-hydroxy-2-propylphenoxy)pentyloxy]-3,4-dihydro-2H-1-benzopyran-2-carboxylic acid n-octyl ester, m.p. 59-67° C; $[\alpha]_D^{25}$ +0.77° (c, 1.034, $CHCl_3$).

## Example 7

Preparation of racemic 6-acetyl-7-[5-(4-acetyl-3-hydroxy-2-propylphenoxy)pentyloxy]-3,4-dihydro-2H-1-benzopyran-2-carboxylic acid n-decyl ester

A 1 L flask equipped with a mechanical stirrer, a Dean-Stark trap, a reflux condenser and a calcium chloride drying tube was charged with 30.0 g (0.060 mole) of racemic 6-acetyl-7-[5-(4-acetyl-3-hydroxy-2-propylphenoxy)pentyloxy]-3,4-dihydro-2H-1-benzopyran-2-carboxylic acid, 9.5 g (0.060 mole) of 1-decanol, 0.3 g (0.0015 mole) of p-toluenesulfonic acid monohydrate and 300 ml of toluene. The mixture was heated at reflux temperature for 2 hours with the removal of water. After cooling to room temperature, the solution was washed with 2 x 50 ml = 100 ml of brine, 3 x 50 ml = 150 ml of saturated sodium bicarbonate solution, and 2 x 50 ml = 100 ml of brine. The organic phase was dried over anhydrous sodium sulfate, filtered and evaporated to dryness at 45° C (70 mm) and at 45° C (0.1 mm) to give 37.9 g of yellow oil. This solid was dissolved in 100 ml of warm ether, diluted with 300 ml of hexanes, seeded and placed in the refrigerator (12° C) overnight to effect crystallization. The resulting white solids were filtered and dried overnight in a vacuum oven at room temperature (1mm) to give 32.6 of white solid racemic 6-acetyl-7-[5-(4-acetyl-3-hydroxy-2-propylphenoxy)pentyloxy]-3,4-dihydro-2H-1-benzopyran-2-carboxylic acid n-decyl ester, m.p. 59-61° C.

## Example 8

### Oral Formulation

(Tablet)

| Ingredients: | mg/tablet |
|---|---|
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 250.0 mg |
| Lactose | 50.0 mg |
| Starch | 25.0 mg |
| Polyvinylpyrrolidone | 2.5 mg |
| Magnesium stearate | 2.5 mg |
| Total weight | 330.0 mg |

Procedure:

Granulate a mixture of the powdered ingredients with the polyvinylpyrrolidone in solution and press into a tablet. Then coat the tablet by spray coating or by rotating the tablet in a coating pan with the following coating solution:

| | Wt % |
|---|---|
| Hydroxypropyl methylcellulosepthalate | 6 |
| Alcohol | 47 |
| Methylene chloride | 47 |
| Total weight | 100 % |

The resulting coated tablets are resistant to gastric fluid but dissolve in intestinal fluid.

Example 9

Oral Formulation

(Soft gelatin capsule)

| Ingredients: | mg/capsule |
|---|---|
| Sodium salt of racemic 6-acetyl-7-[5-(4-acetyl-3-hydroxy-2-propylphenoxy)pentyloxy]-3,4-dihydro-2H-1-benzopyran-2-carboxylic acid n-octyl ester | 261.0* mg |
| Triglycerides (medium chain length) | 229.0 mg |
| Sodium silicates | 10.0 mg |
| Total weight | 500.0 mg |

* Equivalent to 250 mg of free acid

Procedure:

The ingredients are formed into a suspension and loaded into a soft gelatin capsule. The capsule is coated to make it resistant to gastric fluid but dissolves in intestinal fluid.

## Example 10

### Oral Formulation

(Beadlets)

| Ingredients: | mg/dose |
|---|---|
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 250 mg |
| Polyvinylpyrrolidone | 25 mg |
| Starch | 50 mg |
| Total weight | 325 mg |

Procedure:

The beadlets are prepared by wet granulation and then coated with the below polymeric solution by spraying on the surface of the beadlets in a rotating coating pan.

| | Wt. % |
|---|---|
| Polymethacrylic acid polymer | 5.0 |
| Ethyl cellulose | 1.0 |
| Acetone | 10.0 |
| Isopropyl alcohol | 84.0 |
| Total weight | 100.0 % |

The coated beadlets are resistant to gastric fluid but dissolve in intestinal fluid.

Example 11

Oral Formulation

(Tablet)

| Ingredients: | mg/tablet | |
|---|---|---|
| | 100 mg | 600 mg |
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 100 mg | 600 mg |
| Lactose | 30 mg | 100 mg |
| Pregelatinized starch | 4 mg | 25 mg |
| Microcrystalline cellulose | 20 mg | 120 mg |
| Modified starch | 5 mg | 25 mg |
| Magnesium stearate | 1 mg | 8 mg |
| Total weight | 160 mg | 878 mg |

Procedure:

Racemic 6-acetyl-7-[5-(4-acetyl-3-hydroxy-2-propylphenoxy)pentyloxy]-3,4-dihydro-2H-1-benzopyran-2-carboxylic acid n-octyl ester, lactose, pregelatinized starch and microcrystalline cellulose are mixed together and granulated in water, the granulated material is dried at 45-50° C and milled, the modified starch and magnesium stearate are added, and the resulting granulation mixture is pressed into a tablet.

## Example 12

### Oral Formulation

(Tablet)

| Ingredients: | mg/tablet | |
|---|---|---|
| | 100 mg | 600 mg |
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 100 mg | 600 mg |
| Lactose | 40 mg | 200 mg |
| Polyvinylpyrrolidone | 4 mg | 24 mg |
| Modified starch | 4 mg | 24 mg |
| Magnesium stearate | 1 mg | 6 mg |
| Total weight | 149 mg | 854 mg |

Procedure:

Racemic 6-acetyl-7-[5-(4-acetyl-3-hydroxy-2-propylphenoxy)pentyloxy]-3,4-dihydro-2H-1-benzopyran-2-carboxylic acid n-octyl ester, lactose and modified starch are mixed together and granulated with polyvinylpyrrolidone in water or alcohol, the granulated material is dried at 45-50°C and milled, the magnesium stearate is added, and the resulting granulation mixture is pressed into a tablet.

## Example 13

### Oral Formulation

(Capsule)

| Ingredients: | mg/capsule | |
|---|---|---|
| | 100 mg | 600 mg |
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 100 mg | 600 mg |
| Lactose* | 50 mg | -- |
| Pregelatinized starch** | 5 mg | 30 mg |
| Talc | 4 mg | 24 mg |
| Magnesium stearate | 1 mg | 6 mg |
| Total weight | 160 mg | 660 mg |

* Can be substituted by dicalcium phosphate dihydrate, mannitol or sorbitol
** Can be substituted by hydroxypropylmethylcellulose, gelatin or silicates

Procedure:

Racemic 6-acetyl-7-[5-(4-acetyl-3-hydroxy-2-propylphenoxy)pentyloxy]-3,4-dihydro-2H-1-benzopyran-2-carboxylic acid n-octyl ester, lactose and pregelatinized starch are mixed together and granulated in water, the granulated material is dried at 45-50° C and milled, the talc and magnesium stearate are added, and the resulting mixture is loaded into a soft gelatin or liquid capsule.

Example 14

Parenteral Formulation

| Ingredients: | mg/ml |
|---|---|
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 10 mg |
| Mannitol* | 20 mg |
| Sodium hydroxide and/or hydrochlorid acid to adjust pH | q.s. |
| Water (for injection), q.s. to make | 1.0 ml |
| Total volume | 1.0 ml |

* Can be substituted by lactose

Procedure:

Racemic 6-acetyl-7-[5-(4-acetyl-3-hydroxy-2-propylphenoxy)pentyloxy]-3,4-dihydro-2H-1-benzopyran-2-carboxylic acid n-octyl ester and mannitol are dissolved in the water and the pH is adjusted. The solution is then lyophilized in a suitable container (removing the water).

## Example 15

### Parenteral Formulation

| Ingredients: | Percent by Weight |
| --- | --- |
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 1.0% |
| Benzyl alcohol* | 1.0% |
| Propylene glycol** | 10.0% |
| Emulphor** | 5.0% |
| Phosphate buffer, q.s. to pH | 7.5 |
| Water (for injection), q.s. to make | 100% |

* Can be substituted by thimerosal, phenol, methyl and propyl parabens, benzothonium chloride or chlorobutanol

** Can be substituted by polyethylene glycols, alcohol, dimethylacetamide, glycerine, povidone, lecithin, sorbitan monooleate and trioleate

Procedure:

The phosphate buffer is dissolved in a portion of the water, the benzyl alcohol, propylene glycol and emulphor are added with stirring in the buffered solution, racemic 6-acetyl-7-[5-(4-acetyl-3-hydroxy-2-propylphenoxy)pentyloxy]3,4-dihydro-2H-1-benzopyran-2-carboxylic acid n-octyl ester is dissolved in the solution, the pH is adjusted and water is added to the required amount, and the resulting solution is filled into a suitable container.

## Example 16

## Rectal Formulation

(Suppository)

| Ingredients: | mg/suppository | |
|---|---|---|
| | 100 mg | 1000 mg |
| Racemic 6-acetyl-7-[5-(4-acetyl-3-hydroxy-2-propylphenoxy)pentyloxy]-3,4-dihydro-2H-1-benzopyran-2-carboxylic acid n-octyl ester | 100 mg | 1000 mg |
| Suppository base*, q.s. to make | 1000 mg | 2000 mg |
| Total weight | 1000 mg | 2000 mg |

* Contains semi-synthetic glycerides, blend of triglycerides such as Witepsol H15 of Dynamit Nobel Chemicals, and polyethylene glycols 400 and 4000, individually or in combination as desired

Procedure:

The racemic 6-acetyl-7-[5-(4-acetyl-3-hydroxy-2-propylphenoxy)pentyloxy]-3,4-dihydro-2H-1-benzopyran-2-carboxylic acid n-octyl ester is dispersed in the molten suppository base and the resulting mass is filled into a suppository shell and sealed.

Example 17

Rectal Formulation

(Suppository)

| Ingredients: | mg/suppository | |
|---|---|---|
| | 100 mg | 1000 mg |
| Sodium salt of racemic 6-acetyl-7-[5-(4-acetyl-3-hydroxy-2-propylphenoxy)pentyloxy]-3,4-dihydro-2H-1-benzopyran-2-carboxylic acid n-octyl ester | 100 mg | 1000 mg |
| Sorbitan trioleate | 100 mg | 600 mg |
| Cocoa butter, q.s. to make | 1000 mg | 2000 mg |
| Total weight | 1000 mg | 2000 mg |

Procedure:

The cocoa butter is melted and the sodium salt of racemic 6-acetyl-7-[5-(4-acetyl-3-hydroxy-2-propylphenoxy)pentyloxy]-3,4-dihydro-2H-1-benzopyran-2-carboxylic acid n-octyl ester and sorbitan trioleate are dispersed in it, and the mass is filled into a suppository shell and sealed.

## Example 18

### Rectal Formulation

(Enema)

| Ingredients: | mg/enema | |
|---|---|---|
| | 100 mg | 1000 mg |
| Sodium salt of racemic 6-acetyl-7-[5-(4-acetyl-3-hydroxy-2-propylphenoxy)pentyloxy]-3,4-dihydro-2H-1-benzopyran-2-carboxylic acid n-octyl ester | 100 mg | 1000 mg |
| Polyethylene glycol 400 | 10 ml | 15 ml |
| Water for injection, q.s. to make | 120 ml | 180 ml |
| Total volume | 120 ml | 180 ml |

Procedure:

The sodium salt of racemic 6-acetyl-7-[5-(4-acetyl-3-hydroxy-2-propylphenoxy)pentyloxy]-3,4-dihydro-2H-1-benzopyran-2-carboxylic acid n-octyl ester and polyethylene glycol 400 are premixed and dissolved in the water for injection, and the solution is loaded into an appropriate squeezable plastic container from which it can be dispersed.

## Claims

1. Compounds of the general formula

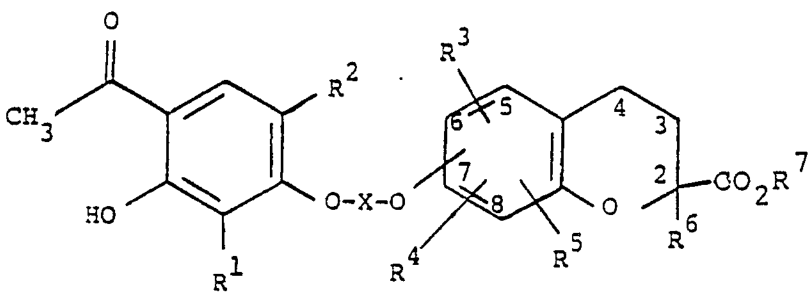

I

wherein $R^1$ is hydrogen or lower alkyl, $R^2$ is hydrogen or halogen, $R^3$, $R^4$ and $R^5$, independently, are hydrogen, acyl or lower alkyl, provided that only one of $R^3$, $R^4$ and $R^5$ can be acyl, $R^6$ is hydrogen or lower alkyl, $R^7$ is higher alkyl or benzyl and X is $(C_{3-7})$-alkylene,
and enantiomers thereof.

2. Compounds in accordance with claim 1, wherein $R^1$ is lower alkyl, $R^2$ is hydrogen, $R^3$ is acyl and $R^4$, $R^5$ and $R^6$ are hydrogen.

3. Compounds in acordance with claim 1 or 2, wherein $R^1$ is propyl, $R^3$ is acetyl in 6-position and X is $(C_{4-6})$-alkylene.

4. Racemic 6-acetyl-7-[5-(4-acetyl-3-hydroxy-2-propylphenoxy)pentyloxy]-3,4-dihydro-2H-1-benzopyran-2-carboxylic acid n-octyl ester, or an enantiomer thereof.

5. Racemic 6-acetyl-7-[5-(4-acetyl-3-hydroxy-2-propylphenoxy)pentyloxy]-3,4-dihydro-2H-1-benzopyran-2-carboxylic acid n-decyl ester, or a enantiomer thereof,
racemic 6-acetyl-7-[5-(4-acetyl-3-hydroxy-2-propylphenoxy)pentyloxy]-3,4-dihydro-2H-1-benzopyran-2-carboxylic acid n-dodecyl ester, or an enantiomer thereof,
racemic 6-acetyl-7-[5-(4-acetyl-3-hydroxy-2-propylphenoxy)pentyloxy]-3,4-dihydro-2H-1-benzopyran-2-carboxylic acid n-hexadecyl ester, or an enantiomer thereof.
racemic 6-acetyl-7-[5-(4-acetyl-3-hydroxy-2-propylphenoxy)pentyloxy]-3,4-dihydro-2H-1-benzopyran-2-carboxylic acid benzyl ester, or an enantiomer thereof,
(R)-(-)-6-acetyl-7-[5-(4-acetyl-3-hydroxy-2-propylphenoxy)pentyloxy]-3,4-dihydro-2H-1-benzopyran-2-carboxylic acid n-octyl ester or
(S)-(+)-6-acetyl-7-[5-(4-acetyl-3-hydroxy-2-propylphenoxy)pentyloxy]-3,4-dihydro-2H-1-benzopyran-2-carboxylic acid n-octyl ester.

6. A compound in accordance with any one of claims 1 to 5 for use as a therapeutically active substance.

7. A compound in accordance with any one of claims 1 to 5 for use in the control or prevention of allergic or inflammatory disorders.

8. A process for the manufacture of a compound in accordance with any one of claims 1 to 5 which comprises

a) reacting a compound of the general formula

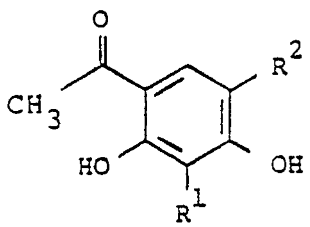 II or 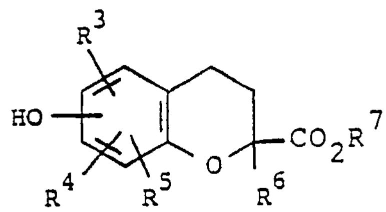 III

wherein $R^1$, $R^2$, $R^3$, $R^4$, $R^5$, $R^6$ and $R^7$ are as in claim 1, with, respectively, a compound of the general formula

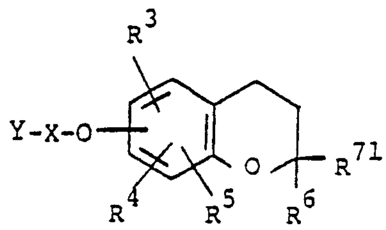 IV and 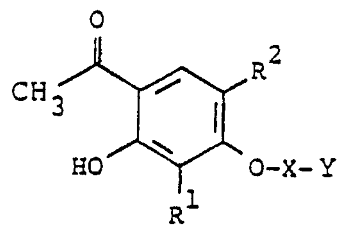 V

wherein $R^1$, $R^2$, $R^3$, $R^4$, $R^5$, $R^6$, $R^7$ and X are as in claim 1 and Y is halogen, alkylsulfonate, aralkylsulfonate or arylsulfonate, or

b) esterifying a compound of the general formula

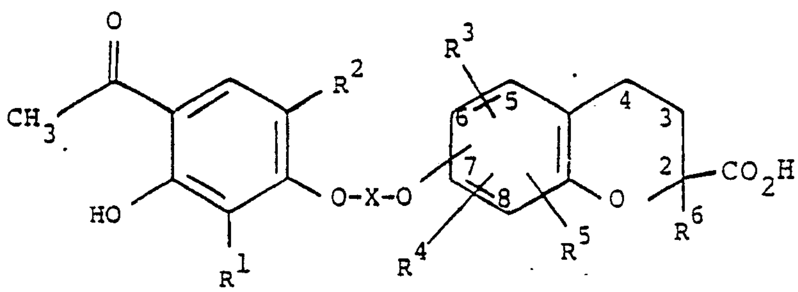 VI

wherein $R^1$, $R^2$, $R^3$, $R^4$, $R^5$, $R^6$ and X are as in claim 1, and

c) if desired, resolving a racemate obtained into the optically active isomers.

9. A medicament containing a compound in accordance with any one of claims 1 to 5 and a therapeutically inert excipient.

10. A medicament for the control or prevention of allergic or inflammatory disorders containing a compound in accordance with any one of claims 1 to 5 and a therapeutically inert excipient.

11. The use of a compound in accordance with any one of claims 1 to 5 in the control or prevention of illnesses.

12. The use of a compound in accordance with any one of claims 1 to 5 in the control or prevention of allergic or inflammatory disorders.

13. The use of a compound in accordance with any one of claims 1 to 5 for the manufacture of a medicament against allergic or inflammatory disorders.

European Patent Office

# PARTIAL EUROPEAN SEARCH REPORT

which under Rule 45 of the European Patent Convention shall be considered, for the purposes of subsequent proceedings, as the European search report

Application number: EP 89101886.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 139 809 (G.D.SEARLE & CO.)<br>* Claims 1,25; page 1; example 35 *<br>-- | 1,6-10,13 | C 07 D 311/66<br>A 61 K 31/35 |
| D,A | EP - A1 - 0 129 906 (F.HOFFMANN-LA ROCHE & CO.)<br>* Claims 1,20-22 *<br>-- | 1,6-10,13 | |
| A | EP - A2/A3 - 0 150 447 (G.D.SEARLE & CO.)<br>* Claims 1,19; page 1, line 35 - page 2,lines 1-2; page 41 *<br>---- | 1,6-10,13 | |

TECHNICAL FIELDS SEARCHED (Int. Cl.4)

C 07 D 311/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10,13

Claims searched incompletely: -

Claims not searched: 11,12

Reason for the limitation of the search: Art. 52(4) EPC; Method for treatment of human or animal body by therapy

| Place of search | Date of completion of the search | Examiner |
|---|---|---|
| VIENNA | 26-04-1989 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1. 03.82